# EUROPEAN PATENT APPLICATION

(11) **EP 4 527 410 A1**
(43) Date of publication of application: **26.03.2025**
(21) Application number: 24201544.4
(22) Date of filing: 20.09.2024
(51) Int. Cl.: A61K 45/06, A61K 38/13, A61K 31/4439

(54) **COMPOSITION FOR INHIBITING CORONAVIRUSES OR ENTEROVIRUSES**

(30) Priority: 22.09.2023 EP 23199067
(71) Applicant: PHILIPPS-UNIVERSITÄT MARBURG, 35037 Marburg (DE); Universität zu Lübeck, 23562 Lübeck (DE)
(72) Inventor: Becker, Stephan, 35043 Marburg (DE); Sauerhering, Lucie, 35043 Marburg (DE); Hilgenfeld, Rolf, 23538 Lübeck (DE)
(74) Representative: Durm Patentanwälte PartG mbB

(57) **Abstract**

The present application relates to a composition for inhibiting the replication and/or release of coronaviruses or enteroviruses, comprising a) a cyclophilin inhibitor, and b) an inhibitor of the 3C protease of enteroviruses or an inhibitor of the 3C-like proteases of coronaviruses. Furthermore, the present application relates to the use of this composition as a medicament.

## Description

The present invention relates to a composition for inhibiting the replication of coronaviruses or enteroviruses as well as this composition for use as a medicament, in particular for treating infections with coronaviruses or enteroviruses.

Inhibitors of the SARS-CoV-2 main protease typically suffer from a lack of stability against degradation by enzymes of the cytochrome P450 family, which is why they must be administered together with inhibitors of these enzymes, such as ritonavir. Thus, the lack of longevity of the inhibitors of the SARS-CoV-2 main protease is a problem.

The problem of the lack of longevity of the inhibitors of the SARS-CoV-2 main protease can be at least partially solved by further improving the inhibitory activity of the protease inhibitors. Combination with ritonavir or related compounds can delay the degradation of the protease inhibitors. The disadvantage of such a combination is that ritonavir and its derivatives are added to many other drugs as stabilizers. This means that drugs containing ritonavir are often contraindicated for COViD-19 therapy.

Thus, the technical problem underlying the present invention is to provide a composition which do not have the disadvantages known from the prior art as mentioned above.

This has been achieved by the subject-matter of the independent claims, wherein preferred embodiments are defined in the dependent claims.

According to the present invention, there is provided a composition for inhibiting the replication and/or release of coronaviruses or enteroviruses, comprising
a) a cyclophilin inhibitor, and
b) an inhibitor of the 3C protease of enteroviruses or an
inhibitor of the 3C-like proteases of coronaviruses.

With this composition, a novel therapeutic approach to inhibit coronaviruses or enteroviruses is provided, whereby different inhibitors are combined to simultaneously inhibit the virus at different points in its replication cycle. With this composition, a synergistic inhibitory effect can be achieved.

The term "inhibiting" as used herein means that the replication and/or release of the viruses is lowered when the composition according to the present invention is used compared to the replication and/or release of the viruses without the use of the composition according to the present invention. The inhibiting effect can by synergistically. The inhibiting effect can be observed in all tissues/cells relevant for the attack of the virus, in particular the effect is observed in tissue/cells of the lung or intestinal tract.

In one embodiment of the composition according to the present invention, the inhibitor of the 3C-like proteases of coronaviruses is selected from the group of the inhibitors of the 3C-like protease of SARS-CoV-2, MERS-CoV and HCoV-229E, in particular the inhibitor of the 3C-like protease of coronaviruses is the compound 13b-K.

The compound 13b-K has the chemical name *tert*-butyl (1-((*S*)-1-(((*S*)-4-(benzylamino)-3,4-dioxo-1-((*S*)-2-oxopyrrolidin-3-yl)butan-2-yl)amino)-3-cyclopropyl-1-oxopropan-2-yl)-2-oxo-1,2-dihydropyridin-3-yl)carbamate. It has the following chemical formula:

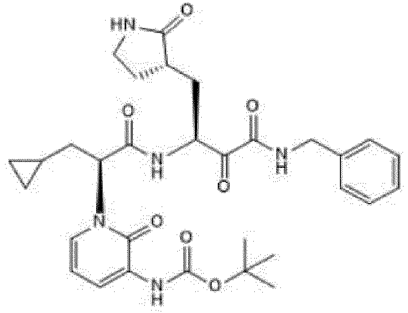

The inhibitor 13b-K is commercially available. For example, it can be purchased from Bio-Techne Corporation.

In another embodiment of the composition according to the present invention the inhibitor of the 3C protease of enteroviruses is selected from the group consisting of inhibitors of the 3C protease of the following enteroviruses: enterovirus A71, coxsackieviruses, poliovirus, enterovirus D68.

In a further embodiment, the cyclophilin inhibitor present in the composition according to the present inventions is selected from the group consisting of cyclosporin A.

Examples of specific combinations of the inhibitors a) and b) of the composition according to the present invention are cyclosporin A (CsA) with 13b-K.

In another embodiment the composition according to the present invention is a medicament, in particular wherein the medicament is adapted for oral application. The medicament contains the composition according to the present invention, i.e. the two above-described compounds a) and b). Additionally, the medicament can contain further ingredients used according to the prior art, for example excipients. When the medicament is for oral application, it can be a tablet. The skilled person knows the compounds as well methods for providing tablets. The present invention is not restricted to a specific tablet form.

A further aspect of the present invention is the composition as defined above for use as a medicament, in particular for use in the treatment of infections caused by coronaviruses and enteroviruses.

The present invention will be described in more detail in the following by reference to cyclosporin A and 13b-K, but it is to be understood that the invention is not restricted thereto but the following information can be adapted to other inhibitors as well.

Cyclophilin inhibitors, in particular cyclosporin A, have been used in hospitals after organ transplants for several decades. Its tolerability has therefore been tried and tested over many years. It has been found that cells that are treated with CsA are put into an antiviral state and can thus defend themselves against various coronaviruses. With the combination with a protease inhibitor (for example 13b-K), which can be specifically directed against the essential protease, an inhibition of virus replication is achieved. The virus is thus inhibited from different sides simultaneously.

This is a novel approach. The present invention combines the antiviral effects of cyclophilin inhibitors (cyclosporin A) and 13b-K (protease inhibitor), whereby surprisingly a synergistic effect of the two substances was found.

To be more specific, as mentioned above, CsA is a cyclophilin A inhibitor used as an immunosuppressant to prevent transplant rejection. CsA can reduce the replication of various coronaviruses, for example SARS-CoV-2 and MERS-CoV *in vitro* and *in vivo.* The antiviral effect is partly due to CsA - induced IFN-λ, which induces the expression of antiviral interferon stimulated genes (ISGs). 13b-K is a coronavirus inhibitor directed against the viral main protease (MPro, also known as 3CLpro, encoded by nsp5), which is essential for processing and maturation of the coronavirus polyproteins. It was also found that 13b-K is able to inhibit SARS-CoV-2 replication. Treatment with CsA and 13b-K alone reduce viral replication or egress by 2-3 log scales. The effect of a combination treatment with both substances (cyclophilin inhibitor and MPro inhibitor) on the replication of HCoV-229E, SARS-CoV-2 or MERS-CoV has been investigated.

First, 13b-K was tested against SARS-CoV-2 and MERS-CoV in fully differentiated human bronchial epithelial cells to confirm the inhibitory properties in most relevant cell culture models. The treatment with 13b-K results in decreased viral replication of both SARS-CoV-2 and MERS-CoV by 2 log scales. Next, CsA was combined with 13b-K during infection of bronchial cells with different SARSCoV-2 variants of concern or MERS-CoV. The combination of both substances leads to a complete block of viral replication and release of all tested betacoronaviruses. Furthermore, it was also found significant reduction of virus induced cytokines in inhibitor-treated and infected cells. These results show that the cytokine storm which is described in severe COVID-19 and could lead to tremendous lung damage is also positively affected by the composition according to the present invention.

The combination treatment described here is able to completely inhibit betacoronaviruses in human-relevant cell culture models. By using two different substance classes, it is possible to counteract the viruses at different important propagation steps. 13b-K directly interferes with the virus replication while the cyclophilin inhibitor enhances cellular defense mechanisms due to an increased IFN-λ expression and ISG induction.

The composition according to the present inventions has several advantages compared with the prior art. First, it has not the disadvantages as discussed above with compositions of the prior art. In particular, the composition has a good longevity and it does not contain substances causing contraindications. Further, there are combined substances already used in the clinic. The replication of various coronaviruses is significantly inhibited.

The invention is further described by the following examples and figures. It is explicitly pointed out that the examples and figures only serve to further illustrate the present invention and they shall not be construed to limit the invention thereto.
Fig. 1 shows the effect of the combination of inhibitors against SARS-CoV-2 variants in Calu3 cells.
Fig. 2 shows pictures of the effect of the combination of inhibitors against MERS-CoV in Calu3 cells.
Fig. 3 shows the effect of the combination of inhibitors against MERS-CoV in Calu3 cells.
Fig. 4 shows pictures of the effect of the combination of inhibitors against MERS-CoV at low concentration in Calu3 cells.
Fig. 5 shows the effect of the combination of inhibitors against MERS-CoV at low dose in Calu3 cells.
Fig. 6 shows the effect of the combination of inhibitors against HCoV-229E in Calu3 cells.
Fig. 7 shows the effect of the combination of inhibitors against HCoV-229E at low concentration in Calu3 cells.
Fig. 8 shows the effect of the combination of inhibitors against HCoV-229E at low concentration in Caco2 cells.

### Examples

Cyclosporin A (CsA) is a cyclophilin A inhibitor used as an immunosuppressant to prevent transplant rejection. CsA can reduce the replication of various coronaviruses (CoVs), for example SARS-CoV-2 and MERS-CoV *in vitro* and *in vivo.* The antiviral effect is partly due to CsA - induced IFN-λ, which induces the expression of antiviral interferon stimulated genes (ISGs). 13b-K is a coronavirus inhibitor directed against the viral main protease (MPro, also known as 3CLpro, encoded by nsp5), which is essential for processing and maturation of the coronavirus polyprotein. It was shown that 13b-K is able to inhibit SARS-CoV-2 replication. Treatment with CsA and 13b-K alone reduce viral replication or egress by 2-3 log scales. It was investigated the effect of a combination treatment with both substances (cyclophilin inhibitor and MPro inhibitor) on the replication of different SARS-CoV-2 variants, HCoV-229E or MERS-CoV.

### Methods

### Corona Virus (CoV) infection

Experiments with MERS-CoV and SARS-CoV-2 were performed under biosafety level 4 conditions at the Institute of Virology, Philipps University of Marburg, Germany. Experiments with HCoV-229E were performed under biosafety level 2 conditions at the Institute of Virology, Philipps University of Marburg, Germany. Calu-3 or Caco2-cells were infected at a multiplicity of infection of 0.1 diluted in DMEM/F12 without fetal calf serum (FCS) at 37°C for 1h. Cells were washed with DMEM/F12 with 10% FCS and supplemented with cyclosporin A (CsA) and 3C/3C-like Protease-Inhibitor 13b-K either alone or in combination (concentrations: 5-10 µM). 24h post infection, cells were processed for quantitative PCR (Luna universal qPCR Mastermix, NEB, USA) and the supernatant was harvested for virus titration as described previously.

First, it was analyzed the effect of 13b-K on SARS-CoV-2 (BavPat) and MERS-CoV infection in bronchial epithelial cells (Calu3, A+B). 13b-K is able to reduce the release of both viruses in a dose-dependent manner. Next, primary, bronchial epithelial cells of three different human donors were cultivated under air- liquid-interface conditions for several weeks and the cultures were infected with both CoVs (C). When the cells were treated with 13b-K it was found reduced viral replication of both viruses indicating that 13b-K is able to also reduce CoV-infections under nearly natural conditions in fully differentiated epithelial cells.

The following results shown in the Figures were obtained.

Since viral replication of CoVs was not fully abolished after treatment with CsA or 13b-K alone, both inhibitors were combined for increased efficacy. Therefore, Calu3 cells were infected with different variants of SARS-CoV-2 and the cells were treated with a combination of CsA and 13b-K. 24h pi the amount of viral RNA was quantified in infected cell lysates. In all three tested viruses the combination of CsA and 13b-K reduce viral mRNA amounts by 5-6 log scales. Therefore, it was concluded that a combined therapy of direct and indirect inhibitors almost fully suppresses SARS-CoV-2 replication.

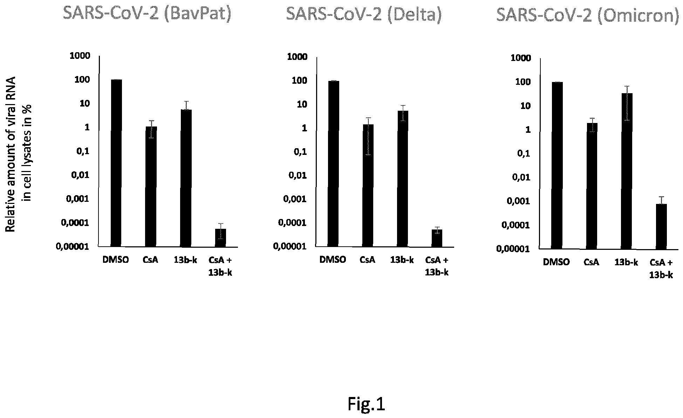

The results are shown in Fig. 1, which shows the effect of the combination of the inhibitors against SARS-CoV-2 variants in Calu3 cells. Calu3 cells are human bronchial epithelial cells. It can be taken from Fig. 1 that the combined therapy of direct and indirect inhibitors almost fully suppresses the SARS-Cov-2 replication.

To validate these finding also in the context of other highly pathogenic CoVs the experiments were repeated in MERS-CoV-infected Calu3 cells.

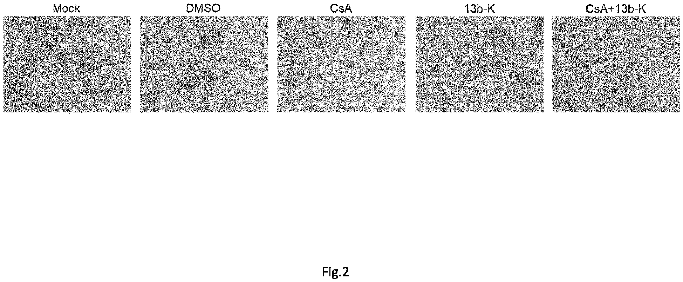

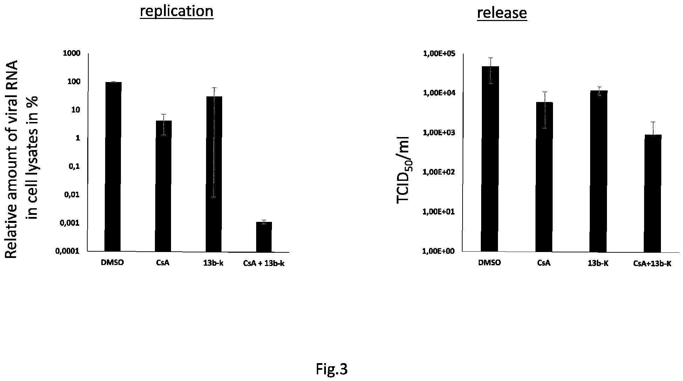

The MERS-CoV infection of Calu3 cells results in formation of foci. This allows to also analyze foci formation as a further readout. When the infected cells were treated with either CSA or 13b-K (10 µm each) a reduction of foci formation was seen, but only the combination of CsA and 13b-K completely rescued the infected cells of virus induced morphological changes (see Fig. 2) Next, the viral replication and release was quantified (see Fig. 3). Also, in MERS-CoV infection the combination of a cyclophilin inhibitor and 13b-K was able to almost completely reduce CoV infection (5 log scales). The results are shown in Fig. 2 and 3.

Fig. 2 shows photographs of the effect of the combination of inhibitors against MERS-CoV in Calu3 cells. For the experiments the human bronchial epithelial cells Calu3 have been used. It can be taken from the photographs that the combined therapy of the two inhibitors according to the present invention almost fully suppresses the MERS-CoV replication.

Fig. 3 shows the effect of the combination of inhibitors against MERS-CoV in Calu3 cells. As can be taken from Fig. 3, the combined use of the two inhibitors according to the present invention almost fully suppresses the MERS-CoV replication and also the release.

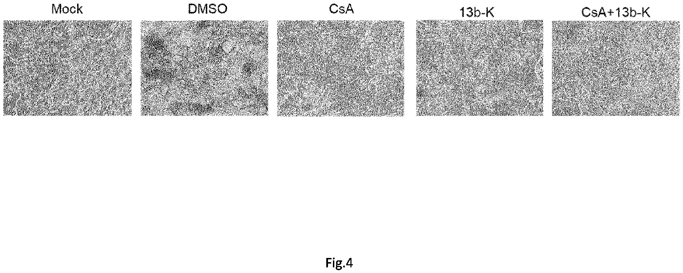

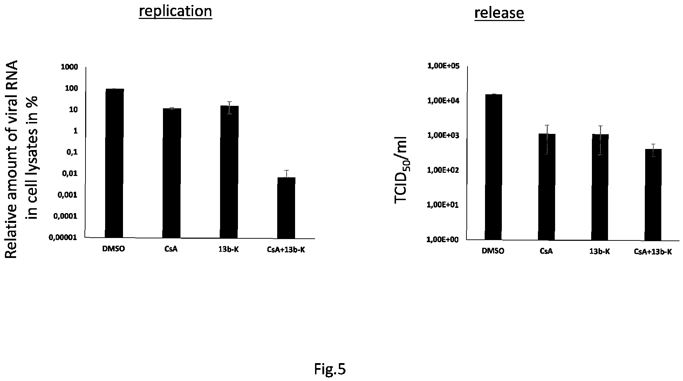

Next, the cells were treated with a lower dose of all inhibitors. CsA (5 µM) can counteract virus-induced foci formation but the inhibitory effect is less compared to former results indicating a dose-depended inhibition. The treatment with 13b-K (5 µM) alone leads to similar results. Nevertheless, the combination of cyclophilin inhibitor and 13b-K using lower dose could clearly diminished foci formation and viral replication by 4 log scales. That is, the combined use of direct and indirect inhibitors almost fully suppresses MERS-CoV foci formation, replication and release. The results are shown in Fig. 4 and 5.

Fig. 4 shows photographs of the effect of the combination of inhibitors against MERS-CoV at low concentration of 5 µM in Calu3 cells. It can be taken from the photographs that the combined therapy of the two inhibitors according to the present invention almost fully suppresses the MERS-CoV replication even at a lower dose of 5 µM.

Fig. 5 shows the effect of the combination of the inhibitors against MERS-CoV at low concentration of 5 µM in Calu3 cells. It can be taken from Fig. 5 that also a lower dose of the combination of the two inhibitors (here CsA on the one hand and 13b-K on the other hand) reduces the MERS replication (4 logs) and had also an effect on the viral release (about 1 log) .

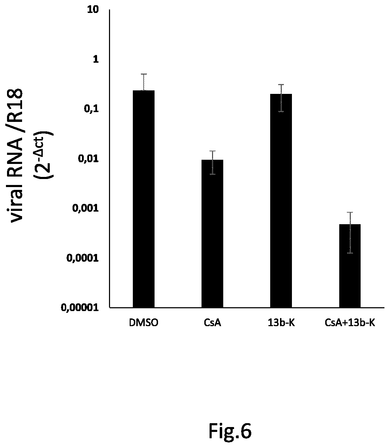

Fig. 6 shows the effect of the combination of the two inhibitors against HCoV-229E in Calu3 cells. HCoC-229E causes respiratory and intestinal diseases in humans. The experiments were again carried out with Calu3 cells. As can be taken from Fig. 6, the cyclophilin inhibitors CsA can reduce the viral replication about 1 log. The combination of the cyclophilin inhibitors with 13b-K has a significantly greater effect than the use of the cyclophilin inhibitors alone.

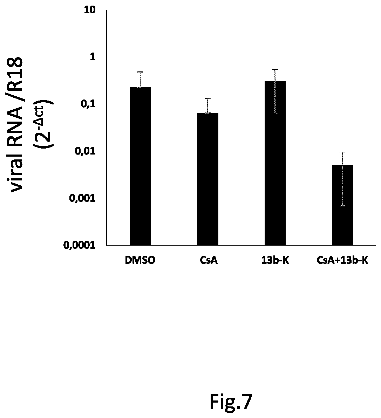

Fig. 7 shows the effect of the combination of the inhibitors against HCoV-229E at low concentration in Calu3 cells. The concentration used was 5 µM. From Fig. 7 it can be derived that the cyclophilin inhibitor CsA can reduce the viral replication about ½ log. The combination of the cyclophilin inhibitor with 13b-K has a significantly greater effect than the use of the cyclophilin inhibitor alone.

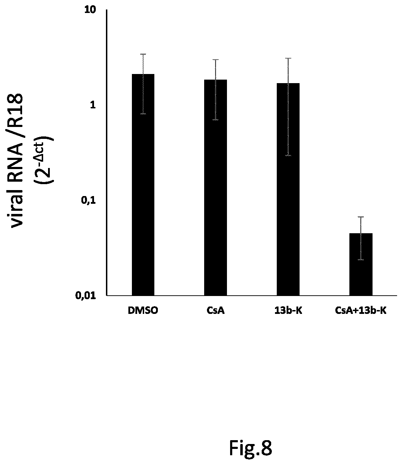

Fig. 8 shows the effect of the combination of the inhibitors against HCoV-229E at low concentration in Caco2 cells, which are human intestinal epithelia cells. The used concentration was 5 pm. The cyclophilin inhibitor alone as well as 13b-K alone have no effect. However, when both compounds are combined, a significant effect can be observed.

### Results

### 1) Combined therapy counteracted HCoV-229E infection

According to the present invention, both inhibitors were combined. First, the combination was tested against HCoV-229E, which is low pathogenic representative of the coronaviruses. The infection was investigated in both Calu3 cells and Caco-2 cells, as 229E has the capacity to infect both the respiratory and the intestinal tract. The combination of both groups of active substances led to a more pronounced reduction in viral replication when they were tested in combination compared to when they were used alone in all tested cell types.

### 2) Combined use suppressed different variants of SARS-CoV-2

Next, Calu3 cells were infected with different variants of SARS-CoV-2 and treated the cells with a combination of CsA and 13b-K. After 24h the amount of viral RNA was quantified in infected cell lysates. In all three tested viruses the combination of CsA and 13b-K reduce viral mRNA amounts by 5-6 log scales.

Therefore, it can be concluded that a combined use of direct and indirect inhibitors almost fully suppresses SARS-CoV-2 replication.

### 3) Combined use diminished MERS-CoV infection

To validate these findings also in the context of other highly pathogenic CoVs the experiments were repeated in MERS-CoV-infected Calu3 cells. The MERS-CoV infection of Calu3 cells results in formation of foci. This allows to also analyse foci formation as a further readout. When the infected cells were treated with either CsA or 13b-K a reduction of foci formation was observed, but only the combination of CsA and 13b-K completely rescued the infected cells of virus induced morphological changes. Next, the viral replication and release was quantified. Also, in MERS-CoV infection the combination of a cyclophilin inhibitor and 13b-K was able to almost completely reduce CoV infection (5 log scales).

The cells were also treated with a lower dose of all inhibitors. CsA can counteract virus-induced foci formation but the inhibitory effect is less compared to former results indicating a dose-depended inhibition. The treatment with 13b-K alone leads to similar results. Nevertheless, the combination of cyclophilin inhibitor and 13b-K using lower dose could clearly diminished foci formation and viral replication by 4 log scales.

Combined use of direct and indirect inhibitors almost fully suppresses MERS-CoV foci formation, replication and release.

### Conclusion

The combined use described here is able to completely inhibit different CoVs in human-relevant cell culture models. By using two different substance classes, it is possible to counteract the viruses at different important propagation steps. 13b-K directly interferes with the virus replication while the cyclophilin inhibitor enhance cellular defense mechanisms due to an increased IFN-λ expression and ISG induction.

## Claims

1. Composition for inhibiting the replication and/or release of coronaviruses or enteroviruses, comprising
a) a cyclophilin inhibitor, and
b) an inhibitor of the 3C protease of enteroviruses or an inhibitor of the 3C-like proteases of coronaviruses.

2. The composition according to claim 1, wherein the inhibitor of the 3C-like proteases of coronaviruses is selected from the group of the inhibitors of the 3C-like protease of SARS-CoV-2, MERS-CoV and HCoV-229E.

3. The composition according to claim 1 and 2, wherein the inhibitor of the 3C-like protease of coronaviruses is the compound 13b-K.

4. The composition according to claim 1, wherein the inhibitor of the 3C protease of enteroviruses is selected from the group consisting of inhibitors of the 3C protease of the following enteroviruses: enterovirus A71, coxsackieviruses, poliovirus, enterovirus D68.

5. The composition according to any of the preceding claims, wherein the cyclophilin inhibitor is selected from the group consisting of cyclosporin A.

6. The composition according to any of the preceding claims, wherein the composition is a medicament.

7. The composition according to claim 1, wherein the medicament is adapted for oral application.

8. Composition as defined in any of claims 1 to 7 for use as a medicament.

9. The composition according to any of claims 1-7 for use in the treatment of infections caused by coronaviruses and enteroviruses.
